# EUROPEAN PATENT SPECIFICATION

(11) **EP 2 793 790 B1**
(45) Date of publication and mention of the grant of the patent: **02.10.2019**
(21) Application number: 12805903.7
(22) Date of filing: 17.12.2012
(51) Int. Cl.: A61F 13/56, A61F 13/47

(54) **ABSORBENT ARTICLE COMPRISING A FRAGRANCE OR ODOR CONTROL COMPOSITION**
ABSORBIERENDER ARTIKEL ENTHALTEND EIN PARFÜM ODER EINE KOMPOSITION ZUR GERUCHSKONTROLLE
ARTICLE ABSORBANT COMPRENANT UN PARFUM OU UNE COMPOSITION DE CONTRÔLE DES ODEURS

(30) Priority: 20.12.2011 US 201161577693 P
(43) Date of publication of application: 29.10.2014
(73) Proprietor: The Procter & Gamble Company, Cincinnati, OH 45202 (US)
(72) Inventor: CAPUTI, Mariangela, I-65126 Pescara (IT); BELLUCCI, Remo, I-65010 SPOLTORE (Pescara) (IT); D'ERCOLE, Luigia, I-65015 Montesilvano (IT); DENTI, Federica, 65812 Schwalbach am Taunus (DE); DELGADO, Liliana, Singapore, 138547 (SG); SILVA SEGARRA, Vanessa, Liberty Township, Ohio 45011 (US)
(74) Representative: P&G Patent Germany
(86) International application number: PCT/US2012/070068
(87) International publication number: WO 2013/096185

(56) References cited:
- EP-A2- 1 077 053
- GB-A- 2 390 546
- US-A- 5 342 333
- US-A1- 2004 067 214
- US-A1- 2005 113 779
- US-A1- 2007 287 973

## Description

### FIELD OF THE INVENTION

The present invention relates to an absorbent article comprising a fastening adhesive and a liquid fragrance or odor control composition in non overlapping patterns.

### BACKGROUND OF THE INVENTION

Absorbent articles for personal hygiene which during use are attached to the underwear of the user are known in the art. Typical examples include sanitary napkins, pantiliners and incontinence pads. Such articles are commonly used to absorb and retain bodily fluids and other exudates excreted by the human body, such as urine and menses. Typically, such exudates are perceived as malodorous and offensive. Therefore, methods and materials for controlling and reducing malodors in absorbent articles have been developed. Fragrance compositions have been widely used for this purpose in absorbent articles. Other compositions which may not properly be defined as "fragrance" compositions per se (because that they do not per se possess a pleasant odor) have been used to reduce the perception of malodors. These are generally called "odor control compositions". Such compositions usually contain, sometimes along with conventional perfume ingredients, ingredients which are able to chemically react with the malodorant molecules released from the body fluids (such as ammonia) thus neutralizing the source of the malodor, and/or ingredients which are able to interact with nose receptors so that their perception of the malodorant molecules is reduced. Examples of such compositions are described in WO2007/113778 A2.

GB 2390546 A, US 2004/067214 A1 and US 5342333 A disclose absorbent articles including a liquid fragrance or odor control composition.

Fragrance and odor control compositions are usually formed by blends of organic compounds including aldehydes, chetons, esters, ethers, alcohols, essential oils, solvents and the like. It is well known that such organic compounds, when employed in absorbent articles, during storage of the absorbent articles, tend to migrate toward the backsheet and trough it into the fastening adhesive. As a result the properties of the fastening adhesive can be altered to the point that the adhesive is not effective anymore in keeping the product in place attached to the undergarments or/and in that the fastening adhesive leaves residues on the undergarments once the product is removed after use.

This problem is known in the art, it has been partially solved by immobilizing the perfume on solid substrates so to prevent their migration (see for example US4237591). Still this solution adds complexity to the manufacturing process as it requires the use of "inert" materials like substrates and/or porous solids which are more difficult to handle than a simple liquid fragrance or odor control composition.

Therefore a need still exist for an absorbent article comprising a liquid fragrance or odor control composition and a fastening adhesive which is easy to manufacture using standard equipment and wherein the fastening adhesive is not affected, even upon prolonged storage, by the migration of the fragrance or odor control composition.

### SUMMARY OF THE INVENTION

The present invention relates to an absorbent article and to a method for manufacturing an absorbent article, as defined in the appended independent claims. Further embodiments of the absorbent article are defined in the dependent claims.

The absorbent article of the present invention exhibits no or very little migration of the fragrance or odor control composition components into the fastening adhesive (PFA) and consequently no degradation of the PFA.

### BRIEF DESCRIPTION OF THE DRAWINGS

FIG. 1 is a IR spectrum of a fastening adhesive in an aged absorbent article wherein the fastening adhesive and the liquid fragrance or odor control composition are applied in patterns which do not overlap at all.
FIG. 2 is a IR spectrum of a fastening adhesive in an aged absorbent article wherein the fastening adhesive and the liquid fragrance or odor control composition are applied in patterns which entirely overlap.
FIG. 3 is a representation of an absorbent article wherein the fastening adhesive and the liquid fragrance or odor control composition are applied in patterns which do not overlap at all, and wherein these patterns are represented as projected onto the garment facing surface of the article.

### DETAILED DESCRIPTION OF THE INVENTION

"Absorbent article" refers to devices that absorb and contain body exudates, such as urine, menses, and feces. The term "disposable" is used herein to describe absorbent articles which are not intended to be laundered or otherwise restored or reused as an absorbent article after a single use. Examples of absorbent articles include diapers, toddler training pants, adult incontinence garments, and feminine hygiene garments such as sanitary napkins, pantiliners, interlabial devices, hemorrhoid pads, and the like. Absorbent articles according to the present invention are selected from sanitary napkins, incontinence pads and pantyliners.

Absorbent articles and components thereof according to the present invention, including the topsheet, backsheet, absorbent core, and any individual layers of these components, have a body-facing surface and a garment-facing surface. As used herein, "body-facing surface" means that surface of the article or component which is intended to be worn toward or adjacent to the body of the wearer, while the "garment-facing surface" is on the opposite side and is intended to be worn toward or placed adjacent to the wearer's undergarments when the disposable absorbent article is worn.

In general, the absorbent articles of the present invention typically comprise a topsheet, a backsheet, and (with the exception of thin pantyliners which are not meant to absorb fluids but just to provide a clean feeling to the panties) an absorbent core disposed between the topsheet and backsheet.

The topsheet of the absorbent article is preferably compliant, soft feeling, and nonirritating to the wearers skin and hair. Further, the topsheet is liquid pervious, permitting liquids (e.g., menses and/or urine) to readily penetrate through its thickness. A suitable topsheet may be manufactured from a wide range of materials such as woven and nonwoven materials (e.g., a nonwoven web of fibers); polymeric materials such as apertured formed thermoplastic films, apertured plastic films, and hydroformed thermoplastic films; porous foams; reticulated foams; reticulated thermoplastic films; and thermoplastic scrims. Suitable woven and nonwoven materials can be comprised of natural fibers (e.g., wood or cotton fibers), synthetic fibers (e.g., polymeric fibers such as polyester, polypropylene, or polyethylene fibers) or from a combination of natural and synthetic fibers. When the topsheet comprises a nonwoven web, the web may be manufactured by a wide number of known techniques. For example, the web may be spunbonded, carded, wet-laid, melt-blown, hydroentangled, combinations of the above, or the like.

The backsheet is impervious to liquids (e.g., menses and/or urine) and is preferably manufactured from a thin plastic film, although other flexible liquid impervious materials may also be used. As used herein, the term "flexible" refers to materials which are compliant and will readily conform to the general shape and contours of the human body. The backsheet prevents the exudates absorbed and contained in the absorbent core from wetting articles which contact the absorbent article such as bedsheets, pants, pajamas and undergarments. The backsheet can also be vapor permeable ("breathable"), while remaining fluid impermeable. The backsheet may comprise a woven or nonwoven material, polymeric films such as thermoplastic films of polyethylene or polypropylene, or composite materials such as a film-coated nonwoven material.

The backsheet and the topsheet can positioned adjacent the garment surface and the body surface, respectively, of the absorbent core. The absorbent core can be joined with the topsheet, the backsheet, or both in any manner as is known by attachment means such as those well known in the art. Embodiments of the present invention are envisioned wherein portions of the entire absorbent core are unattached to either the topsheet, the backsheet, or both.

The absorbent core can be formed from any of the materials well known to those of ordinary skill in the art. Examples of such materials include multiple plies of creped cellulose wadding, fluffed cellulose fibers, wood pulp fibers also known as airfelt, textile fibers, a blend of fibers, a mass or batt of fibers, airlaid webs of fibers, a web of polymeric fibers, and a blend of polymeric fibers. Other suitable absorbent core materials include absorbent foams such as polyurethane foams or high internal phase emulsion ("HIPE") foams. Suitable HIPE foams are disclosed in US 5,550,167, US 5,387,207, US 5,352,711, and US 5,331,015.

For some absorbent articles, the absorbent core can be relatively thin, less than about 5 mm in thickness, or less than about 3 mm, or less than about 1 mm in thickness. Thickness can be determined by measuring the thickness at the midpoint along the longitudinal centerline of the pad by any means known in the art for doing while under a uniform pressure of 1.72 kPa.

The absorbent core can comprise superabsorbent materials such as absorbent gelling materials (AGM), including AGM fibers, as is known in the art. The absorbent core can therefore constitute a layer comprising superabsorbent material.

According to an embodiment of the present invention, the superabsorbent material for the absorbent core can be selected among polyacrylate based materials, typically in particle form, as described in U.S. Patent Application No. 2008/0172017 A1. The polyacrylate based materials incorporated in the absorbent articles of the present invention are polyelectrolytes with a multiplicity of anionic functional groups, typically carboxyl groups. In certain embodiments, the polyacrylate based materials can comprise polyacrylates, polymethacrylates, and derivatives thereof, such as for example polyacrylate sodium, polymethacrylate sodium, polyacrylate potassium, polymethacrylate potassium, starch grafted polyacrylate, starch grafted polymethacrylate, polyvinyl alcohol grafted polyacrylate, polyvinyl alcohol grafted polymethacrylate, cellulose grafted polyacrylate, cellulose grafted polymethacrylate, and the like. In an embodiment of the present invention, the absorbent gelling material can be a crosslinked, partially neutralized polyacrylate.

The polyelectrolytes which provide the polyacrylate based materials incorporated in the absorbent articles of the present invention can be made from polymerizable, unsaturated, acid-containing monomers. Such monomers include the olefinically unsaturated acids and anhydrides which contain at least one carbon to carbon olefinic double bond. More specifically, these monomers can be selected from olefinically unsaturated carboxylic acids and acid anhydrides, olefinically unsaturated sulfonic acids, and mixtures thereof.

Polyacrylate based materials, typically partially neutralized polymers, are commonly incorporated in absorbent articles and are known as superabsorbent polymers or superabsorbents, and are crosslinked. The polyacrylate material has neutralized, typically with sodium, carboxylate groups hanging off the main polymer chain. In contact with water, the sodium detaches and goes in solution, leaving only carboxyl ions. Being negatively charged, these ions repel one another so that the polymer unwinds and absorbs more and more water, which is instead attracted by the carboxyl ions, as further carboxyl ions become available. The hydrogen in water is trapped by the polyacrylate due to the atomic bonds associated with the polarity forces between the atoms. The cross-links, which bridge different polymer chains, lead to a three dimensional structure, which upon liquid absorption constitutes the swollen gel.

According to an embodiment of the present invention, the absorbent gelling material which can be comprised in the absorbent core can be selected among the polyacrylate based polymers described in the European Patent Application EP 05023061.4, filed on 21 Oct. 2005 in the name of The Procter and Gamble Company. As explained in the referenced application, polyacrylate based materials being very slightly crosslinked, or substantially not crosslinked at all, incorporated in absorbent articles for the absorption of proteinaceous or serous body fluids such as for example menses, blood, plasma, vaginal secretions, and also mucus or milk, but particularly menses or blood, provide an improved absorption and retention capacity for such body fluids, and an improved absorption rate as well, compared to traditional crosslinked superabsorbents.

According to the above referenced application, a measure of the degree of crosslinking of a polyacrylate based polymer can be expressed in terms of the soluble or extractable fraction of the polymer. As it is known in the art, lower molecular weight polymer chains can be solubilized, or extracted, from the polymer in certain conditions, and represent said soluble or extractable fraction of the polymer itself. Generally, the extractable fraction can be considered to be inversely proportional to the degree of crosslinking, that is, the higher the degree of crosslinking, the lower the fraction, since a greater proportion of the polymer mass is actually incorporated into the polymer network. Such polyacrylate based polymer which can be incorporated in an absorbent article for absorption of proteinaceous or serous body fluids, particularly menses, has an extractable fraction of at least about 30% by weight, between about 30% and about 80% by weight, or between about 32% and about 70% by weight, evaluated according to the Extractables test method described in the PCT Patent Application WO 07/047598. Alternatively, said polyacrylate based materials can have a retention capacity of at least about 30 g/g, at least about 35 g/g, or at least about 40 g/g, evaluated according to the Centrifuge Retention Capacity test described in the PCT Patent Application WO 07/047598.

The absorbent gelling materials can be typically used in the form of discrete particles. Such absorbent gelling materials can be of any desired shape, e.g., spherical or semi-spherical, cubic, rod-like polyhedral, etc. Shapes having a large greatest dimension/smallest dimension ratio, like needles and flakes, are also contemplated for use herein. Agglomerates of absorbent gelling material particles may also be used.

Absorbent cores may include a core wrap i.e. a thin layer of fluid pervious material (usually a tissue paper or a think nonwoven layer) which wraps the core in order to preserve its integrity during manufacturing of the article and during its use.

The absorbent article of the present invention can comprise other additional components, for example between the topsheet and absorbent core, such as a secondary topsheet or acquisition layer. The secondary topsheet or acquisition layer can comprise a tissue layer or a nonwoven, such as carded resin-bonded nonwovens, embossed carded resin-bonded nonwovens, high-loft carded resin-bonded nonwovens, carded through-air-bonded nonwovens, carded thermo-bonded nonwovens, spunbonded nonwovens, and the like. A variety of fibers can be used in the secondary topsheet or acquisition layer, including natural fibers, e.g. wood pulp, cotton, wool, and the like, as well as biodegradeable fibers, such as polylactic acid fibers, and synthetic fibers such as polyolefins (e.g., polyethylene and polypropylene), polyesters, polyamides, synthetic cellulosics (e.g., RAYON®, Lyocell), cellulose acetate, bicomponent fibers, and blends thereof. The basis weight of the secondary topsheet or acquisition layer can vary depending upon the desired application.

The absorbent article can comprise further components such as side cuffs, typically found in diapers, or side wings or side flaps, typically found in sanitary napkins.

The absorbent articles herein are preferably disposable after a single use and are usually commercialized in packages comprising multiple units which in some cases can be individually wrapped.

Suitable components for the fragrance or odor control compositions include fragrance components and reactive components. Fragrance components are typically used in the field of perfumery to provide a composition with an aesthetically pleasing scent. Reactive components include components that can react with malodors, such as ammonia-based malodors or sulphur-based malodors (i.e. "malodor reactive components"), and components that mask malodors and/or react with receptors of the nose to block the perception of malodor by the nose of a consumer (i.e. "malodor masking components"). Suitable reactive components are described, for example, in US 2008/0071238 A1 and WO 2007/113778 A2.

In terms of reactive components, those reacting with ammonia or malodorants sulphur compounds like thiols can be very effective in the present invention. Ammonia and thiols are two common components of malodor associated with the absorption bodily fluids, such as menses or urine. For example, ammonia is typically present in high amounts in absorbent products used for urine absorption due to degradation of urea.

Aldehydes and/or ketones can react with Ammonia and its derivatives to form imines (according to the so-called Schiff base reaction) or via Michael addition reactions.

Aldehydes and/or ketones can also react with thiols forming thioacetals or via Michael addition.

In all cases the resulting compounds are non volatile and therefore essentially odorless.

Many aldehydes and ketones capable the reactions described above have an unpleasant and/or too intense odor that can be disturbing to human nose and/or they are very volatile and so not stable in the product. Therefore, selected aldehydes and/or ketones for controlling such malodors are used. Examples of suitable aldehydes and ketones for controlling malodour are those aldehydes and ketones that are able to react with amine compounds and thiol compounds and have not unpleasant odor. Suitable aldehydes include hexyl cinnamic aldehyde, alpha-amylcinnamic aldehyde, p-anisaldehyde, 4-Formyl-2-methoxyphenyl 2-methylpropanoate, benzaldehyde, cinnamic aldehyde, cuminic aldehyde, decanal, p-t-butyl-alpha-methyldihydrocinnamaldehyde, 4-hydroxy-3-methoxycinnamaldehyde, 2-phenyl-3-(2-furyl)prop-2-enal, vanillin isobutyrate, ethyl vanillin acetate, vanillin acetate, cyclamen aldehyde, heptanal, lauryl aldehyde, nonanal, octanal, phenylacetaldehyde, phenyl propyl aldehyde, vanillin, salycil aldehyde, cytral, 2,4-dihydroxy-3-methylbenzaldehyde, 2-hydroxy-4-methylbenzaldehyde, 5-methyl salicylic aldehydes, 4-nitrobenzaldehyde, o-nitrobenzaldehyde, 5-ethyl-2-thiophenecarbaldehyde, 5-methyl-2-thiophenecarboxaldehyde, 2-thiophenecarbaldehyde, asaronaldehyde, 5-(hydroxymethyl)-2-furaldehyde, 2-benzofurancarboxaldehyde, 2,3,4-trimethoxybenzaldehyde, protocatechualdehyde, heliotropine, 4-ethoxy-3-methoxy benzaldehyde, 3,4,5-trimethoxybenzaldehyde, 3-hydroxybenzaldehyde, o-methoxycinnamaldehyde, 3,5-dimethoxy-4-hydroxycinnamaldehyde, 2,8-dithianon-4-3n-4-carboxaldehyde, sorbinaldehyde, 2,4-heptadienal, 2,4-decadienal, 2,4-nonadienal, 2,4-nonadienal, (E,E)-,2,4-octadien-1-al, 2,4-octadienal, 2,4-dodecadienal, 2,4-undecadienal, 2,4-tridecadien-1-al, 2-trans-4-cis-7-cis-tridecatrienal, piperonylidene propionaldehyde, 2-methyl-3-(2-furyl)acrolein, 2,4-pentadienal, 2-furfurylidene butyraldehyde, 3-(2-furyl)acrolein, pyruvaldehyde, ethanedial or mixtures thereof.

Suitable aldehydes can also be selected from hexyl cinnamic aldehyde, decanal, 4-formyl-2-methoxyphenyl 2-methylpropanoate, 4-hydroxy-3-methoxycinnamaldehyde, 3,5-dimethoxy-4-hydroxycinnamaldehyde, 2-phenyl-3-(2-furyl)prop-2-enal, ethyl vanillin acetate, vanillin isobutyrate, vanillin acetate, asaronaldehyde, or mixtures thereof.

Suitable aldehydes can also be selected from hexyl cinnamic aldehyde, 4-hydroxy-3-methoxycinnamaldehyde, decanal, or mixtures thereof.

Suitable ketones include 1-(2,6,6-trimethyl-1-cyclohexenyl)pent-1-en-3-one, 4-(2,6,6-trimethyl-1-cyclohexen-1-yl)-3-Buten-2-one, 4-(2,6,6-trimethyl-2-cyclohexen-1-yl)-3-buten-2-one (isomers), 5-(2,6,6-Trimethyl-2-cyclohexen-1-yl) 4-penten-3-one, (E)-4-(2,2-dimethyl-6-methylidenecyclohexyl)but-3-en-2-one, laevo-carvone, or mixtures thereof.

Preferably, the malodor reactive component is selected from the group consisting of hexyl cinnamic aldehyde, alpha-amylcinnamic aldehyde, p-anisaldehyde, benzaldehyde, cinnamic aldehyde, cuminic aldehyde, decanal, cyclamen aldehyde, p-t-butyl-alpha-methyldihydrocinnamaldehyde, 4-hydroxy-3-methoxycinnamaldehyde, vanillin isobutyrate, 2-phenyl-3-(2-furyl)prop-2-enal, ethyl vanillin acetate, vanillin acetate, heptanal, lauryl aldehyde, nonanal, octanal, phenylacetaldehyde, phenyl propyl aldehyde, vanillin, salycil aldehyde, cytral, 2,4-dihydroxy-3-methylbenzaldehyde, 2-hydroxy-4-methylbenzaldehyde, 5-methyl salicylic aldehydes, 4-nitrobenzaldehyde, o-nitrobenzaldehyde, 5-ethyl-2-thiophenecarbaldehyde, 5-methyl-2-thiophenecarboxaldehyde, 2-thiophenecarbaldehyde, asaronaldehyde, 5-(hydroxymethyl)-2-furaldehyde, 2-benzofurancarboxaldehyde, 2,3,4-trimethoxybenzaldehyde, protocatechualdehyde, heliotropine, 4-ethoxy-3-methoxy benzaldehyde, 3,4,5-trimethoxybenzaldehyde, 3-hydroxybenzaldehyde, o-methoxycinnamaldehyde, 3,5-dimethoxy-4-hydroxycinnamaldehyde, 2,8-dithianon-4-3n-4-carboxaldehyde, sorbinaldehyde, 2,4-heptadienal, 2,4-decadienal, 2,4-nonadienal, 2,4-nonadienal, (E,E)- 2,4-octadien-1-al, 2,4-octadienal, 2,4-dodecadienal, 2,4-undecadienal, 2,4-tridecadien-1-al, 2-trans-4-cis-7-cis-tridecatrienal, piperonylidene propionaldehyde, 2-methyl-3-(2-furyl)acrolein, 2,4-pentadienal, 2-furfurylidene butyraldehyde, 3-(2-furyl)acrolein, pyruvaldehyde, ethanedial, Laevo-Carvone, 1-(2,6,6-trimethyl-1-cyclohexenyl)pent-1-en-3-one, 4-(2,6,6-trimethyl-1-cyclohexen-1-yl)-3-Buten-2-one, 4-(2,6,6-trimethyl-2-cyclohexen-1-yl)-3-buten-2-one (isomers), 5-(2,6,6-Trimethyl-2-cyclohexen-1-yl) 4-penten-3-one, (E)-4-(2,2-dimethyl-6-methylidenecyclohexyl)but-3-en-2-one, and mixtures thereof.

Other components suitable herein are components that mask the malodors or react with receptors of the nose. The components that mask the malodor tend to be volatile materials that modify the vapor pressure of the malodour, thereby reducing the impression of the malodour. The components that mask the malodor can also do so by inhibiting the receptors of the nose. When used, these materials may significantly reduce the capability for the nose to detect the malodors. The nose blocking is possible due to the volatile nature of the materials selected, which are released from the absorbent article and are then inhaled into the nose of a consumer, generally within somewhat close range of the absorbent article, e.g. within about 0 to 10 meters of the article by normal breathing (although this should in no way be intended to limit the scope of the invention). The blocking of the nose receptors is, of course, only temporary. Suitable malodor masking components include menthol, menthyl acetate, menthyl lactate, 1-(2,6,6-trimethyl-1-cyclohexenyl)pent-1-en-3-one, 4-(2,6,6-trimethyl-1-cyclohexen-1-yl)-3-Buten-2-one, 4-(2,6,6-trimethyl-2-cyclohexen-1-yl)-3-buten-2-one (isomers), 5-(2,6,6-Trimethyl-2-cyclohexen-l-y1) 4-penten-3-one, (E)-4-(2,2-dimethyl-6-methylidenecyclohexyl)but-3-en-2-one, isomenthyl acetate, isomenthyl propionate, isomenthyl isobutyrate, isomenthyl propionate, isomenthyl butyrate, camphor, p-menthane, limonene, eucalyptol, cresol, linalool, tetra-hydrolinalool, myrcenol, tetra hydromyrcenol, di-hydromyrcenol, myrcene, cytronellol, cytronellyil derivatives, geraniol, geranyl derivatives, linalyl acetate, mugetanol, eugenol, jasmal, terpineol, pinanol, cedrene, damascone, beta pinene, cineole and its derivatives, nonadienol, ethylhexanal, octanol acetate, methyl furfural, terpinene, thujene, amylacetate, benzylacetate, camphene, citronellal, dihydrocumarin, dy hydromyrcenyl acetate, geraniol, geranial, isoamylacetate, ethyl, and/or triethyl acetate, para-cresol, para-cymene, methyl abietate, methyl dihydro jasmonate, hexyl-2-methyl butyrate, benzyl acetate, laevo carvone , hexyl-2-methyl butyrate , eucalyptus, phenyl ethyl alcohol and mixtures thereof. The materials also include their isomeric forms, diastereomers and enantiomers. Advantageously, in general, the above materials have only a very slight inherent odour but show a high degree of malodour masking and/or nose receptor blocking.

Preferably, the malodor masking component is selected from the group consisting of menthol, menthyl acetate, menthyl lactate, 1-(2,6,6-trimethyl-1-cyclohexenyl)pent-1-en-3-one, 4-(2,6,6-trimethyl-1-cyclohexen-1-yl)-3-Buten-2-one, 4-(2,6,6-trimethyl-2-cyclohexen-1-yl)-3-buten-2-one (isomers), 5-(2,6,6-Trimethyl-2-cyclohexen-1-yl) 4-penten-3-one, (E)-4-(2,2-dimethyl-6-methylidenecyclohexyl)but-3-en-2-one, isomenthyl acetate, isomenthyl propionate, isomenthyl isobutyrate, isomenthyl propionate, isomenthyl butyrate, camphor, p-menthane, limonene, eucalyptol, cresol, linalool, tetra-hydrolinalool, myrcenol, tetra hydromyrcenol, di-hydromyrcenol, myrcene, cytronellol, cytronellyil derivatives, geraniol, geranyl derivatives, linalyl acetate, mugetanol, eugenol, jasmal, terpineol, pinanol, cedrene, damascone, beta pinene, cineole and its derivatives, nonadienol, ethylhexanal, octanol acetate, methyl furfural, terpinene, thujene, amylacetate, benzylacetate, camphene, citronellal, dihydrocumarin, dy hydromyrcenyl acetate, geraniol, geranial, isoamylacetate, ethyl, and/or triethyl acetate, para-cresol, para-cymene, methyl abietate, methyl dihydro jasmonate, hexyl-2-methyl butyrate, benzyl acetate, laevo carvone , hexyl-2-methyl butyrate , eucalyptus, phenyl ethyl alcohol, and mixtures thereof.

The components of the fragrance or odor control composition can also include fragrance components that impart an aesthetically pleasing odor character to the mixture. Suitable fragrance components which can be used include limonene, eucalyptol, cresol, linalool, tetra-hydrolinalool, myrcenol, tetra hydromyrcenol, di-hydromyrcenol, myrcene, cytronellol, cytronellyil derivatives, geraniol, geranyl derivatives, linalyl acetate, mugetanol, eugenol, jasmal, terpineol, pinanol, cedrene, damascone, beta pinene, cineole and its derivatives, nonadienol, ethylhexanal, octanol acetate, methyl furfural, terpinene, thujene, amylacetate, benzylacetate, camphene, citronellal, di-hydrocumarin, di-hydromyrcenyl acetate, geraniol, geranial, encalyptus, isoamylacetate, ethyl, and /or triethyl acetate, para-cresol and para-cymene, benzyl-benzoate, isopropyl myristate, methyl abietate, ethanol, isopropanol, diethylene glycol monoethyl ether, glycerol, propylene glycol, 1,2-butylene glycol, dipropylene glycol, 2-methyl-2,4-pentanediol, diethyl phthalate, triethyl citrate, diethyl sebacate.

It may be that, for certain components, the same component can be considered both a malodor reactive component, a malodor masking component, and/or a fragrance component.

The fragrance or odor control compositions can be applied in a variety of ways, and in a variety of patterns, to the absorbent article using conventional low viscous fluid application equipment which is well known to the skilled person such as spray, droplets or beads applicators. Such applicators allow to form any application pattern like stripes, circles, dots, drops, geometric figures, stars, decorative figures, irregular shapes, and the like. Also patterned applications are helpful because they allow a precise application so that it is easier to avoid contact with the glue which connects the various layers of the absorbent article.

The fragrance or odor control compositions is typically disposed in the absorbent article in an amount of from about 1 to about 500 milligrams per absorbent article, from about 3 to about 200 milligrams per absorbent article or from about 4 to about 150 milligrams per absorbent article. In some embodiments where the absorbent article is selected from a sanitary napkin or a pantyliner the amount can be from about 4 to about 100 mg, in some embodiments where the absorbent article is an incontinence device the amount can be from about 30 to about 300 milligrams per absorbent article.

The backsheet typically forms the garment-facing surface of the absorbent article on which the panty fastening adhesive is placed. Panty-fastening adhesives (PFA) can comprise any adhesive or glue used in the art for such purposes. These adhesives typically are pressure sensitive and remain tacky well below their application temperature.

Prior to use of the absorbent article, the areas being coated with PFA are typically protected from contamination and from adhering to another surface where this is not desired by a protective cover means such as a silicone coated release paper, a plastic film or any other easily removable cover. The protective cover means can be provided as a single piece or in a multitude of pieces, e.g. to cover the individual adhesive areas. It also can perform other functions such as provide individualised packaging for the article or provide a disposal function. Any commercially available release paper or film may be used. Suitable examples include BL 30 MG-A SILOX EI/O, BL 30 MG-A SILOX 4 P/O available from Akrosil Corporation, and M&W films available from Gronau in Germany, under the code X-5432.

The PFA may be applied to the garment-facing surface of the absorbent article, typically the backsheet and/or the wings using any one of methods well known in the art for this purpose such as slot coating, spraying and roll printing.

One method of applying the PFA to the garment-facing surface of the absorbent article is the direct coating on the backsheet; another method is printing the PFA onto a release paper, which is then pressed onto the garment-facing surface of the absorbent article. Thereby the PFA is transferred from the release paper to the garment-facing surface of the absorbent article. Such a procedure is described in EP 788,338.

It may be desirable that the PFA has a surface coverage on the backsheet of the article from 10 to 99%, or from 10 to 95%, or from 10 to 60% or from 15 to 50%.

The liquid fragrance or odor control composition in the present invention is applied on or within a layer between the topsheet layer and the backsheet layer of said absorbent article. This means that, since the absorbent article is constituted by a series of layers, the liquid fragrance or odor control composition is applied onto one of the surfaces of these layers. Alternatively, if one of the layers allows it (because for example is a thick fibrous layer such as an absorbent core), the layer can be cut in two along a plane substantially parallel to the garment facing surface of the article and the liquid fragrance or odor control composition can be applied on one of the two surfaces resulting from the cut and then the layer can be re-joined as a single layer.

We also mentioned that the liquid fragrance or odor control composition is applied in a pattern. The fragrance or odor control composition is applied onto the surface of application with any possible application pattern: such as for example stripes, circles, dots, drops, geometric figures, stars, decorative figures, irregular shapes, and the like. In some cases it is possible that the liquid fragrance or odor control composition is applied on more than one layer within the article. In that case, for the purpose of the present invention, the "pattern" of application of the liquid fragrance or odor control composition will be considered as the combination of the various patterns projected on a plane parallel to garment facing surface of the article in a flattened configuration.

Similarly, also the PFA is applied on the backsheet of the absorbent article in a pattern which can be selected from any possible application pattern such as for example stripes, circles, dots, geometric figures, stars, decorative figures, irregular shapes, and the like.

In the present invention the application pattern of the fragrance or odor control composition and the application pattern of the PFA do not overlap for more than for 3% of the total surface of the backsheet of the article when the absorbent article is in a flattened configuration and said patterns are seen along a direction perpendicular to the garment facing surface of the article.

For "pattern of application" of a material on a surface we indicate the parts of that surface which are in contact with the material, in this case the fragrance or odor control composition and the fastening adhesive respectively.

For "to overlap" it is meant "to be in the optical path" of an observer which observes along a direction perpendicular to said garment facing surface of the article (like the moon overlaps the sun in a solar eclipse).

For "total surface of the backsheet" it is intended the total surface of the backsheet of the absorbent article including the wings if present.

In some embodiments of the present invention the application pattern of the fragrance or odor control composition and the application pattern of the PFA do not overlap for more than for 3% of the total surface of the article or more than 1% or more than 0.1%.

In another embodiment the application pattern of the fragrance or odor control composition and the application pattern of the PFA do not overlap at all.

It is believed that among the components of the liquid fragrance or odor control composition, some esters components are more problematic than others when they migrate through the layers of the article and reach the fluid impervious backsheet and the PFA applied on its garment facing surface, both because they migrate more effectively and because they have a more detrimental effect on the properties of the adhesive.

The migration of these components of the fragrance or odor control composition may alter the characteristics of the backsheet and of the PFA glue. In particular the PFA tends to lose adherence with the backsheet so that undesirable residues of glue could be left onto the panties after usage of the article. In other cases the PFA may simply lose its function so that the absorbent article is not properly kept in place during use.

Esters are often used as solvents in fragrance and odor control compositions. In absorbent articles made according to the present invention the migration problem is extremely reduced, so that even fragrances or odor control compositions using a certain amount of ester based ingredients can be used, but in general, to further reduce migration risk, it will be preferred to use fragrance or odor control composition with a controlled content of esters.

The liquid fragrance or odor control composition to be used in the absorbent articles of the present invention comprise from 5 to 50%wt or from 10%wt to 40%wt or from 10%wt to 30%wt of one or more esters selected from: menthyl acetate, menthyl lactate, menthyl propionate, menthyl butyrate, cis-3-hexenyl acetate, methyl-dihydrojasmonate, methyl jasmonate, hexyl iso-butyrate, linalyl acetate, benzyl acetate, phenyl ethyl acetate; and less than 5%wt or less than 2%wt or less than 1%wt of other esters.

In some embodiments the liquid fragrance or odor control composition to be used in the absorbent articles of the present invention comprises from 5%wt to 50%wt or from 10%wt to 40%wt or from 10%wt to 30%wt of two or more esters selected from menthyl acetate, menthyl lactate, menthyl propionate, menthyl butyrate, cis-3-hexenyl acetate, methyl-dihydrojasmonate, methyl jasmonate, hexyl iso-butyrate, linalyl acetate, benzyl acetate, phenyl ethyl acetate and less than 5%wt or less than 2%wt or less than 1%wt of other esters.

In a further embodiment the liquid fragrance or odor control composition to be used in the absorbent articles of the present invention comprises from 5%wt to 50%wt or from 10%wt to 40%wt or from 10%wt to 30%wt of a combination of menthyl acetate and of one or both from methyl-dihydrojasmonate and methyl jasmonate. The composition of this embodiment also can optionally comprise one or more esters selected from menthyl lactate, menthyl propionate, menthyl butyrate, cis-3-hexenyl acetate, hexyl iso-butyrate, linalyl acetate, benzyl acetate, phenyl ethyl acetate in an amount such that the total content of menthyl acetate, menthyl lactate, menthyl propionate, menthyl butyrate, cis-3-hexenyl acetate, methyl-dihydrojasmonate, methyl jasmonate, hexyl iso-butyrate, linalyl acetate, benzyl acetate and phenyl ethyl acetate is comprised from 5%wt to 50%wt or from 10%wt to 40%wt or from 10%wt to 30%wt of the total liquid fragrance or odor control composition. In these embodiments the liquid fragrance or odor control composition comprises less than 5%wt or less than 2%wt or less than 1%wt of other esters not included in the list mentioned above.

We have surprisingly found that and confirmed via IR spectroscopy that migration of the esters of the fragrance or odor control composition and in particular of those esters which are more detrimental to the occurs preferentially in the "z" direction i.e. in a direction perpendicular to the plane of the article.

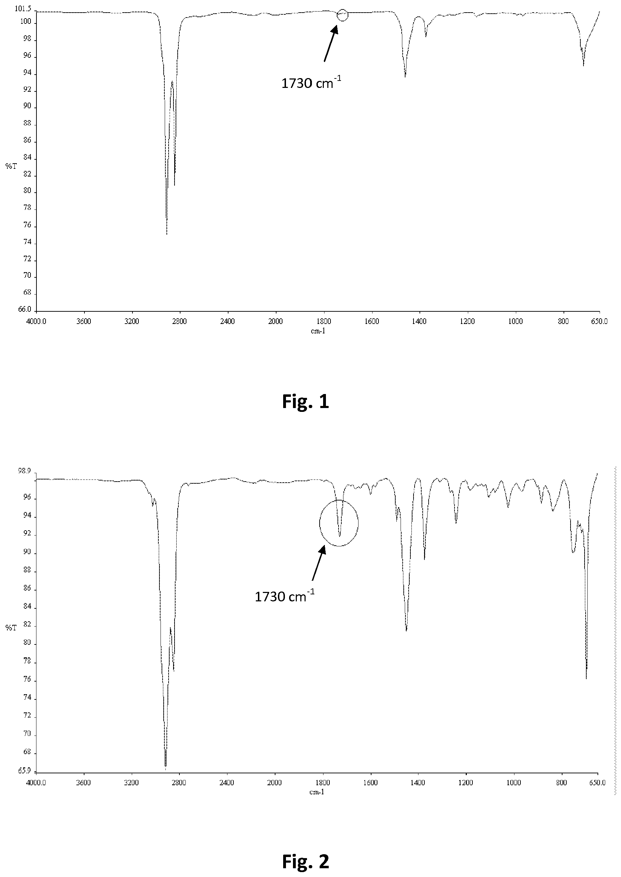

The results obtained are presented in Fig. 1 and 2. Two otherwise equal sanitary napkins of the Type Lines Seta Ultra were prepared, one with non overlapped pattern of fastening adhesive and liquid fragrance or odor control composition (spectrum in Fig. 1) and one with completely overlapped pattern (spectrum in Fig. 2). The articles were stored at room temperature 24 hours, then an IR absorption spectrum of their backsheet surface (with fastening adhesive where present) has been taken directly on the backsheet using a Perkin Elmer Universal ATR sampling equipment (which allows taking a IR spectra of a point in a solid surface). In particular the peak at 1730 cm⁻¹, characteristic for the presence of esters, was monitored. Several points were measured in each article, in particular on the fastening adhesive directly below and around the area overlapped by the pattern of liquid fragrance or odor control composition. The spectra in Fig. 1 and 2 are representative spectra for the respective samples. As it can be seen in Fig. 1 and 2, in the non overlapped sample the ester peak was absent, while in the overlapped sample the ester peak is evident. This confirms that the esters in the liquid fragrance or odor control composition preferentially migrate along a direction z perpendicular to the garment facing surface of the absorbent article.

We have observed that this preferred direction of migration along the z axis is noticeable even when the absorbent articles are stored in a non horizontal position, or even if the articles are folded in package and stored in any orientation. In all cases where the absorbent articles where stored, we surprisingly found that components of the fragrance or odor control composition preferentially migrated along the z direction, i.e. in a direction perpendicular to the plane of the garment facing surface of the article.

Without being bound by theory, it is believed that this preferential direction of migration might explain why articles according to the present invention, having a small or zero overlap between fragrance or odor control composition and PFA demonstrate an improved preservation of the PFA over storage time.

It has also been surprisingly found that, when the absorbent article comprises an absorbent layer which comprises a superabsorbent material such as an absorbent gelling material (AGM) the migration of components from the fragrance or odor control composition toward the backsheet is further reduced. Without being bound by theory it is believed this is due to the fact that the esters are at least partially absorbed by the AGM.

The present invention further encompasses a method for manufacturing an absorbent article selected from a sanitary napkin, an incontinence pad and a pantyliner, the article having a body-facing surface and a garment-facing surface and comprising:
a topsheet layer;
a backsheet layer;
optionally one or more intermediate layers enclosed between topsheet and said backsheet
a fastening adhesive applied on the backsheet garment facing surface.
Absorbent articles of this type are normally made by combining the various layers making up the article on a conveyor belt. The method according to the present invention comprises a step wherein, along the manufacturing line when the layer are combined, a liquid fragrance or odor control composition is applied in liquid form directly on or within a layer of said absorbent article, and wherein said fastening adhesive and said liquid fragrance or odor control composition are applied in patterns which do not overlap when the absorbent article is in a flattened configuration and said patterns are seen along a direction perpendicular to said body facing and garment facing surfaces of the article.

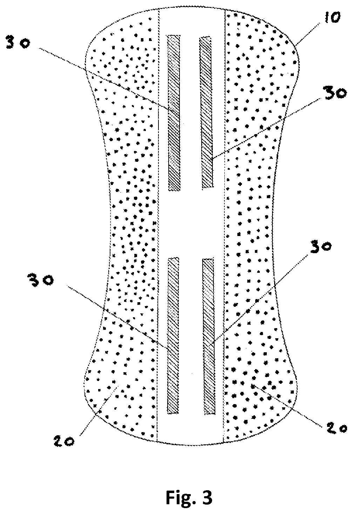

Fig. 3 represents a schematic view of an exemplary sanitary napkin 10 according to the present invention. In the representation of Fig. 3 the patterns of the fastening adhesive and of the liquid fragrance or odor control composition are seen as projected onto the garment facing side of the article even if, as described above, the pattern of fastening adhesive is applied onto the garment facing side of the article while the pattern of liquid fragrance or odor control composition is applied onto the surface of or within one of the other layers making up the article. In Fig. 3 the dotted areas represents the areas where the fastening adhesive is uniformly distributed while the dashed areas 30 represent the areas wherein the pattern of liquid fragrance or odor control composition is uniformly applied. In the example of Fig. 3 the two patterns are not overlapped at all.

The dimensions and values disclosed herein are not to be understood as being strictly limited to the exact numerical values recited. Instead, unless otherwise specified, each such dimension is intended to mean both the recited value and a functionally equivalent range surrounding that value. For example, a dimension disclosed as "40 mm" is intended to mean "about 40 mm."

## Claims

1. An absorbent article selected from a sanitary napkin, an incontinence pad and a pantyliner, having a body-facing surface and a garment-facing surface, said absorbent article comprising:
a topsheet layer;
a backsheet layer;
one or more intermediate layers enclosed between said topsheet and said backsheet;
a fastening adhesive applied on said backsheet garment facing surface; and
a liquid fragrance or odor control composition applied on or within a layer between the topsheet layer and the backsheet layer, wherein said fragrance or odor control composition comprises from 5 to 50%wt of one or more esters selected from: menthyl acetate, menthyl lactate, menthyl propionate, menthyl butyrate, cis-3-hexenyl acetate, methyl-dihydrojasmonate, methyl jasmonate, hexyl iso-butyrate, linalyl acetate, benzyl acetate, phenyl ethyl acetate; and less than 5%wt of other esters not included in the list above,
wherein said fastening adhesive and said liquid fragrance or odor control composition are applied in patterns which do not overlap for more than 3% of the total surface of the backsheet when the absorbent article is in a flattened configuration and said patterns are seen along a direction perpendicular to said body facing and garment facing surfaces of the article.

2. The absorbent article of Claim 1 wherein said fastening adhesive and said liquid fragrance or odor control composition are applied in patterns which do not overlap for more than 1% of the total surface of the backsheet when the absorbent article is in a flattened configuration and said patterns are seen along a direction perpendicular to said body facing and garment facing surfaces of the article.

3. The absorbent article of Claim 1 wherein said fastening adhesive and said liquid fragrance or odor control composition are applied in patterns which do not overlap for more than 0.1% of the total surface of the backsheet when the absorbent article is in a flattened configuration and said patterns are seen along a direction perpendicular to said body facing and garment facing surfaces of the article.

4. The absorbent article of Claim 1 wherein said fastening adhesive and said liquid fragrance or odor control composition are applied in patterns which do not overlap when the absorbent article is in a flattened configuration and said patterns are seen along a direction perpendicular to said body facing and garment facing surfaces of the article.

5. The absorbent article of any preceding claim comprising said one or more intermediate layers wherein at least one of said one or more intermediate layers is an absorbent layer comprising a superabsorbent material.

6. A method for manufacturing an absorbent article selected from a sanitary napkin, an incontinence pad and a pantyliner, said article having a body-facing surface and a garment-facing surface, said absorbent article comprising:
a topsheet layer;
a backsheet layer;
one or more intermediate layers enclosed between said topsheet and said backsheet;
a fastening adhesive applied on said backsheet garment facing surface
said method comprising a step wherein, along the pad manufacturing line, a liquid fragrance or odor control composition is applied in liquid form directly on or within a layer between the topsheet layer and the backsheet layer, and wherein said fastening adhesive and said liquid fragrance or odor control composition are applied in patterns which do not overlap for more than 3% of the total surface of the backsheet when the absorbent article is in a flattened configuration and said patterns are seen along a direction perpendicular to said body facing and garment facing surfaces of the article, wherein said fragrance or odor control composition comprises from 5 to 50%wt of one or more esters selected from: menthyl acetate, menthyl lactate, menthyl propionate, menthyl butyrate, cis-3-hexenyl acetate, methyl-dihydrojasmonate, methyl jasmonate, hexyl iso-butyrate, linalyl acetate, benzyl acetate, phenyl ethyl acetate; and less than 5%wt of other esters not included in the list above.

## Patentansprüche

1. Absorptionsartikel, ausgewählt aus einer Damenbinde, einer Inkontinenzeinlage und einer Slipeinlage, der eine körperseitige Oberfläche und eine bekleidungsseitige Oberfläche aufweist, wobei der Absorptionsartikel Folgendes umfasst:
eine Oberschicht;
eine Unterschicht;
eine oder mehrere Zwischenschichten, die zwischen der Oberschicht und der Unterschicht eingeschlossen sind; einen Befestigungsklebstoff, der auf der bekleidungsseitigen Oberfläche der Unterschicht aufgebracht ist; und eine flüssige Duftstoff- oder Geruchskontrollzusammensetzung, die auf oder innerhalb einer Schicht zwischen der Oberschicht und der Unterschicht aufgebracht ist, wobei die Duftstoff- oder Geruchskontrollzusammensetzung von 5 bis 50 Gew.-% einen oder mehrere Ester umfasst, ausgewählt aus: Menthylacetat, Menthyllactat, Mentylpropionat, Mentylbutyrat, Cis-3-Hexenylacetat, Methyldihydrojasmonat, Methyljasmonat, Hexylisobutyrat, Linalylacetat, Benzylacetat, Phenylethylacetat; und zu weniger als 5 Gew-% andere Ester, die nicht in der obigen Liste enthalten sind,
wobei der Befestigungsklebstoff und die flüssige Duftstoff- oder Geruchskontrollzusammensetzung in Mustern aufgebracht sind, die sich nicht mehr als 3 % der Gesamtoberfläche der Unterschicht überlappen, wenn sich der Absorptionsartikel in einer abgeflachten Konfiguration befindet und die Muster entlang einer Richtung senkrecht zu den körperseitigen und bekleidungsseitigen Oberflächen des Artikels gesehen werden.

2. Absorptionsartikel nach Anspruch 1, wobei der Befestigungsklebstoff und die flüssige Duftstoff- oder Geruchskontrollzusammensetzung in Mustern aufgetragen sind, die sich nicht mehr als 1 % der Gesamtoberfläche der Unterschicht überlappen, wenn sich der Absorptionsartikel in einer abgeflachten Konfiguration befindet und die Muster entlang einer Richtung senkrecht zu den körperseitigen und bekleidungsseitigen Oberflächen des Artikels gesehen werden.

3. Absorptionsartikel nach Anspruch 1, wobei der Befestigungsklebstoff und die flüssige Duftstoff- oder Geruchskontrollzusammensetzung in Mustern aufgetragen sind, die sich nicht mehr als 0,1 % der Gesamtoberfläche der Unterschicht überlappen, wenn sich der Absorptionsartikel in einer abgeflachten Konfiguration befindet und die Muster entlang einer Richtung senkrecht zu den körperseitigen und bekleidungsseitigen Oberflächen des Artikels gesehen werden.

4. Absorptionsartikel nach Anspruch 1, wobei der Befestigungsklebstoff und die flüssige Duftstoff- oder Geruchskontrollzusammensetzung in Mustern aufgebracht sind, die sich nicht überlappen, wenn sich der Absorptionsartikel in einer abgeflachten Konfiguration befindet und die Muster entlang einer Richtung senkrecht zu den körperseitigen und bekleidungsseitigen Oberflächen des Artikels gesehen werden.

5. Absorptionsartikel nach einem der vorstehenden Ansprüche, umfassend die eine oder die mehreren Zwischenschichten, wobei mindestens eine der einen oder der mehreren Zwischenschichten eine Absorptionsschicht ist, die ein Superabsorbermaterial umfasst.

6. Verfahren zur Herstellung eines Absorptionsartikels, ausgewählt aus einer Damenbinde, einer Inkontinenzeinlage und einer Slipeinlage, wobei der Artikel eine körperseitige Oberfläche und eine bekleidungsseitige Oberfläche aufweist, wobei der Absorptionsartikel Folgendes umfasst:
eine Oberschicht;
eine Unterschicht;
eine oder mehrere Zwischenschichten, die zwischen der Oberschicht und der Unterschicht eingeschlossen sind;
einen Befestigungsklebstoff, der auf der bekleidungsseitigen Oberfläche der Unterschicht aufgebracht ist;
wobei das Verfahren einen Schritt umfasst, wobei, entlang der Einlagen-Fertigungslinie, eine flüssige Duftstoff- oder Geruchskontrollzusammensetzung in flüssiger Form direkt auf oder innerhalb einer Schicht zwischen der Oberschicht und der Unterschicht aufgebracht ist, und wobei der Befestigungsklebstoff und die flüssige Duftstoff- oder Geruchskontrollzusammensetzung in Muster aufgebracht sind, die sich nicht mehr als 3 % der Gesamtoberfläche der Unterschicht überlappen, wenn sich der Absorptionsartikel in einer abgeflachten Konfiguration befindet und die Muster entlang einer Richtung senkrecht zu den körperseitigen und bekleidungsseitigen Oberflächen des Artikels betrachtet werden, wobei die Duftstoff oder Geruchskontrollzusammensetzung von 5 bis 50 Gew.-% einen oder mehrere Ester umfasst, ausgewählt aus: Menthylacetat, Menthyllactat, Mentylpropionat, Mentylbutyrat, Cis-3-Hexenylacetat, Methyldihydrojasmonat, Methyljasmonat, Hexylisobutyrat, Linalylacetat, Benzylacetat, Phenylethylacetat; und zu weniger als 5 Gew.-% andere Ester, die nicht in der vorstehenden Liste enthalten sind.

## Revendications

1. Article absorbant choisi parmi une serviette hygiénique, une serviette de protection contre les fuites urinaires et un protège-slip, ayant une surface faisant face au corps et une surface faisant face au vêtement, ledit article absorbant comprenant :
une couche de feuille de dessus ;
une couche de feuille de fond ;
une ou plusieurs couches intermédiaires enfermées entre ladite feuille de dessus et ladite feuille de fond ;
un adhésif de fixation appliqué sur ladite surface faisant face au vêtement de feuille de fond ; et
une composition de parfum ou de régulation des odeurs liquide appliquée sur ou à l'intérieur d'une couche entre la couche de feuille de dessus et la couche de feuille de fond, dans lequel ladite composition de parfum ou de régulation des odeurs comprend de 5 à 50 % en poids d'un ou de plusieurs esters choisis parmi : l'acétate de menthyle, le lactate de menthyle, le propionate de menthyle, le butyrate de menthyle, l'acétate de cis-3-hexényle, le dihydrojasmonate de méthyle, le jasmonate de méthyle, l'iso-butyrate d'hexyle, l'acétate de linalyle, l'acétate de benzyle, le phénylacétate d'éthyle ; et moins de 5 % en poids d'autres esters non inclus dans la liste ci-dessus,
dans lequel ledit adhésif de fixation et ladite composition de parfum ou de régulation des odeurs liquide sont appliqués en motifs qui ne se chevauchent pas sur plus de 3 % de la surface totale de la feuille de fond lorsque l'article absorbant est dans une configuration aplatie et que lesdits motifs sont observés le long d'une direction perpendiculaire auxdites surfaces faisant face au corps et faisant face au vêtement de l'article.

2. Article absorbant selon la revendication 1 dans lequel ledit adhésif de fixation et ladite composition de parfum ou de régulation des odeurs liquide sont appliqués en motifs qui ne se chevauchent pas sur plus de 1 % de la surface totale de la feuille de fond lorsque l'article absorbant est dans une configuration aplatie et que lesdits motifs sont observés le long d'une direction perpendiculaire auxdites surfaces faisant face au corps et faisant face au vêtement de l'article.

3. Article absorbant selon la revendication 1 dans lequel ledit adhésif de fixation et ladite composition de parfum ou de régulation des odeurs liquide sont appliqués en motifs qui ne se chevauchent pas sur plus de 0,1 % de la surface totale de la feuille de fond lorsque l'article absorbant est dans une configuration aplatie et que lesdits motifs sont observés le long d'une direction perpendiculaire auxdites surfaces faisant face au corps et faisant face au vêtement de l'article.

4. Article absorbant selon la revendication 1 dans lequel ledit adhésif de fixation et ladite composition de parfum ou de régulation des odeurs liquide sont appliqués en motifs qui ne se chevauchent pas lorsque l'article absorbant est dans une configuration aplatie et que lesdits motifs sont observés le long d'une direction perpendiculaire auxdites surfaces faisant face au corps et faisant face au vêtement de l'article.

5. Article absorbant selon une quelconque revendication précédente comprenant ladite ou lesdites couches intermédiaires dans lequel au moins l'une parmi ladite ou lesdites couches intermédiaires est une couche absorbante comprenant un matériau superabsorbant.

6. Procédé de fabrication d'un article absorbant choisi parmi une serviette hygiénique, une serviette de protection contre les fuites urinaires et un protège-slip, ledit article ayant une surface faisant face au corps et une surface faisant face au vêtement, ledit article absorbant comprenant :
une couche de feuille de dessus ;
une couche de feuille de fond ;
une ou plusieurs couches intermédiaires enfermées entre ladite feuille de dessus et ladite feuille de fond ;
un adhésif de fixation appliqué sur ladite surface faisant face au vêtement de feuille de fond
ledit procédé comprenant une étape dans laquelle, le long de la ligne de fabrication de la serviette, une composition de parfum ou de régulation des odeurs liquide est appliquée sous forme liquide directement sur ou à l'intérieur d'une couche entre la couche de feuille de dessus et la couche de feuille de fond, et dans laquelle ledit adhésif de fixation et ladite composition de parfum ou de régulation des odeurs liquide sont appliqués en motifs qui ne se chevauchent pas sur plus de 3 % de la surface totale de la feuille de fond lorsque l'article absorbant est dans une configuration aplatie et que lesdits motifs sont observés le long d'une direction perpendiculaire auxdites surfaces faisant face au corps et faisant face au vêtement de l'article, dans laquelle ladite composition de parfum ou de régulation des odeurs comprend de 5 à 50 % en poids d'un ou de plusieurs esters choisis parmi : l'acétate de menthyle, le lactate de menthyle, le propionate de menthyle, le butyrate de menthyle, l'acétate de cis-3-hexényle, le dihydrojasmonate de méthyle, le jasmonate de méthyle, l'iso-butyrate d'hexyle, l'acétate de linalyle, l'acétate de benzyle, le phénylacétate d'éthyle ; et moins de 5 % en poids d'autres esters non inclus dans la liste ci-dessus,
